(19) Europäisches Patentamt
European Patent Office
Office européen des brevets

(11) Veröffentlichungsnummer : **0 061 015**
**B1**

(12) # EUROPÄISCHE PATENTSCHRIFT

(45) Veröffentlichungstag der Patentschrift : **11.04.84**

(51) Int. Cl.³ : **C 07 C 37/20**, C 07 C 39/06, C 07 C 39/08

(21) Anmeldenummer : **82101466.9**

(22) Anmeldetag : **26.02.82**

(54) **Verfahren zur Herstellung eines 2-Alkylphenols.**

(30) Priorität : **05.03.81 DE 3108265**

(43) Veröffentlichungstag der Anmeldung : **29.09.82 Patentblatt 82/39**

(45) Bekanntmachung des Hinweises auf die Patenterteilung : **11.04.84 Patentblatt 84/15**

(84) Benannte Vertragsstaaten : **DE FR GB**

(56) Entgegenhaltungen :
**DD-A- 118 272**
**DE-A- 2 610 374**
**DE-A- 2 716 036**
**US-A- 2 909 568**
**HOUBEN-WEYL, "Methoden der organischen Chemie" 4. Auflage, Band V/1a, Teil 1, 1970, VERLAG GEORG THIEME, Stuttgart Seiten 268 bis 269**

(73) Patentinhaber : **RIEDEL-DE HAEN AKTIENGESELLSCHAFT**
**Wunstorfer Strasse 40**
**D-3016 Seelze 1 (DE)**

(72) Erfinder : **Steinmetz, Arthur, Dr.**
**Goethestrasse 1**
**D-3016 Seelze (DE)**

(74) Vertreter : **Meyer-Dulheuer, Karl-Hermann, Dr. et al**
**HOECHST** Aktiengesellschaft **Zentrale Patentabteilung Postfach 80 03 20**
**D-6230 Frankfurt/Main 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 061 015 B1

Verfahren zur Herstellung eines 2-Alkylphenols

Die Erfindung bezieht sich auf ein Verfahren zur Herstellung eines 2-Alkylphenols durch Reduktion eines 2-Acylphenols in Gegenwart eines metallischen Katalysators bei einem Druck von 10 bis 100 bar, gegebenenfalls in Gegenwart eines inerten Lösemittels.

Es ist bekannt, daß bestimmte Alkylphenole durch Reduktion der entsprechenden Acylphenole mit Hilfe von Wasserstoff in Gegenwart von metallischen Katalysatoren hergestellt werden können. So wurde beispielsweise die Hydrierung von Acylhydrochinon unter Verwendung von Palladium auf Kohle durchgeführt (vgl. US-Patentschriften 2 728 659 und 2 732 300) ; 4,6-Diethylresorcin wurde durch Hydrierung von 4,6-Diacetylresorcin in Gegenwart eines Nickelkatalysators, insbesondere Raney-Nickel, in einer Menge von 0,1 bis 20 Gewichtsprozent innerhalb eines begrenzten Temperaturbereichs von 70 bis 90 °C bei einem Wasserstoffdruck von 20 bis 100 bar erhalten, wobei die Hydrierung auch in Lösung durchführbar ist (vgl. deutsche Offenlegungsschrift 2 610 374). Edelmetallkatalysatoren wie Palladium sind aus Kostengründen nicht universell verwendbar, und Raney-Nickel ist nur begrenzt einsatzfähig, da es gleichzeitig häufig auch Kernhydrierung verursacht.

Aufgabe der Erfindung ist die Schaffung eines Verfahrens zur katalytischen Hydrierung von 2-Acylphenolen, das allgemeiner und in beliebigem Umfang durchführbar ist, mit preiswerten Metallkatalysatoren auskommt und zu hohen Ausbeuten führt.

Die Erfindung betrifft nun ein Verfahren zur Herstellung eines 2-Alkylphenols durch Reduktion eines 2-Acylphenols in Gegenwart eines metallischen Katalysators bei einem Druck von 10 bis 100 bar, gegebenenfalls in Gegenwart eines inerten Lösemittels, das dadurch gekennzeichnet ist, daß bei einer Temperatur von 100 bis 160 °C eine Verbindung der Formel (1)

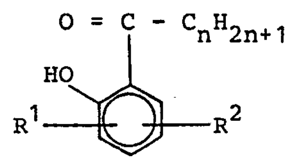

(1)

hydriert wird, in der n eine ganze Zahl von 2 bis 17 darstellt und $R^1$ und $R^2$ gleich oder verschieden sind und jeweils ein Wasserstoffatom, ein Halogenatom, eine Hydroxylgruppe, eine Alkoxygruppe mit 1 bis 16 Kohlenstoffatomen, eine Acylgruppe oder Acyloxygruppe mit jeweils 2 bis 10 Kohlenstoffatomen, eine Dialkylaminogruppe mit insgesamt 2 bis 14 Kohlenstoffatomen oder einen Kohlenwasserstoffrest mit 1 bis 16 Kohlenstoffatomen bedeutet, und als Katalysator Kupfer, Nickel oder Kobalt in einer Menge von 0,5 bis 25 Gewichtsprozent (bezogen auf das 2-Acylphenol) verwendet wird.

Als Ausgangsmaterial für das erfindungsgemäße Verfahren dient ein 2-Acylphenol der Formel (1)

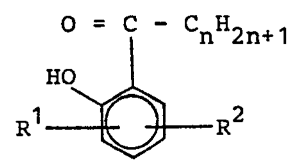

in der n eine ganze Zahl von 2 bis 17, vorzugsweise von 3 bis 7 darstellt und $R^1$ und $R^2$ gleich oder verschieden sind und jeweils ein Wasserstoffatom, ein Halogenatom wie vorzugsweise ein Chlor- oder Bromatom, eine Hydroxylgruppe, eine Alkoxygruppe mit 1 bis 16, vorzugsweise 1 bis 12 Kohlenstoffatomen, eine Acylgruppe oder Acyloxygruppe mit jeweils 2 bis 10, vorzugsweise 4 bis 8 Kohlenstoffatomen, eine Dialkylaminogruppe mit insgesamt 2 bis 14, vorzugsweise 2 bis 8 Kohlenstoffatomen oder einen Kohlenwasserstoffrest mit 1 bis 16, vorzugsweise 1 bis 12 Kohlenstoffatomen bedeutet, wobei letzterer vor allem ein Alkylrest, Phenylrest oder Alkylphenylrest ist.

Die erfindungsgemäße Hydrierung von 2-Acylphenolen zu 2-Alkylphenolen wird bei einer Temperatur von 100 bis 160 °C durchgeführt ; eine Reaktionstemperatur von 135 bis 150 °C ist besonders vorteilhaft. Die Reaktion erfolgt in Gegenwart von gasförmigem Wasserstoff bei einem Druck von 10 bis 100 bar, vorzugsweise 20 bis 60 bar, wobei der Bereich von 30 bis 50 bar am vorteilhaftesten ist.

Es empfiehlt sich, die Umsetzung in Gegenwart eines für die Reaktionskomponenten inerten Lösemittels durchzuführen. Das Lösemittel, das auch ein Gemisch verschiedener Flüssigkeiten sein kann, wird gegebenenfalls in einer Menge von 50 bis 2 000, vorzugsweise 100 bis 1 000 Gewichtsprozent eingesetzt, wobei eine Menge von 200 bis 400 Gewichtsprozent besonders zweckmäßig ist (bezogen auf das 2-Acylphenol). Besonders geeignete Lösemittel sind einwertige oder zweiwertige Alkanole mit 1 bis 4 Kohlenstoffatomen, z. B. Methanol, Ethanol, 1-Propanol, 2-Propanol, 1-Butanol, 2-Butanol, 2-Methylpropanol-(1) 2-Methylpropanol-(2), Ethandiol-(1,2) und Propandiol-(1,2). Ferner eignen sich aprotische Lösemittel, insbesondere Ether wie 1,2-Dimethoxyethan, Tetrahydrofuran und 1,4-Dioxan, gesättigte

Carbonsäureester wie Essigsäureethylester, aromatische Kohlenwasserstoffe wie Benzol, Toluol und Xylol sowie auch Wasser.

Ein wesentliches Merkmal des erfindungsgemäßen Verfahrens ist die Durchführung der Hydrierung in Gegenwart von Kupfer, Nickel oder Kobalt als Katalysator in einer Menge von 0,5 bis 25, vorzugsweise 2 bis 15 Gewichtsprozent, wobei eine Menge von 5 bis 10 Gewichtsprozent besonders bevorzugt ist (bezogen auf das 2-Acylphenol). Der Katalysator wird jeweils unter Berücksichtigung etwaiger Substituenten des 2-Acylphenols ausgewählt.

Eine bevorzugte Ausführungsform des Verfahrens besteht in der Anwendung eines auf einem Träger fixierten Katalysators. Dabei beträgt das Gewichtsverhältnis von Katalysator zu Träger 1 : 0,2 bis 1 : 10, vorzugsweise 1 : 0,5 bis 1 : 2. Diese Ausführungsform hat den Vorteil, daß sich das Reaktionsgemisch in besonders einfacher Weise aufarbeiten läßt. Als Trägermaterial eignen sich Oxide, z. B. Siliciumdioxid, Magnesiumoxid, Aluminiumoxid, Titandioxid, Zirkonoxid und Thoriumoxid, ferner Silikate, z. B. Chrysotil, Actinolit und Crocidolit, sowie Aluminate, z. B. Korund und Bauxit. Hierbei sind Siliciumdioxid (Kieselgur) und Aluminiumoxid (Tonerde) bevorzugt.

Das erfindungsgemäße Verfahren ist kontinuierlich oder diskontinuierlich durchführbar. Üblicherweise werden in einem Autoklav, der mit einem Mischwerkzeug, z. B. einem Rührer, versehen ist, das Ausgangsmaterial, der Katalysator und gegebenenfalls das Lösemittel vorgelegt. Dann wird der Autoklav zur Entfernung des Luftsauerstoffs mit einem Inertgas, vorzugsweise Stickstoff, gespült und mit Wasserstoff gefüllt. Anschließend wird das Gemisch auf Reaktionstemperatur erhitzt und unter ständiger Bewegung, vorzugsweise durch Rühren, und unter dauernder Wasserstoffzufuhr solange auf Reaktionstemperatur gehalten, bis keine Wasserstoffaufnahme mehr erfolgt. Nach Abkühlen des Reaktionsgemisches auf eine Temperatur unterhalb des Siedepunktes des verwendeten Lösemittels, bei Umsetzungen ohne Verwendung eines Lösemittels oberhalb des Schmelzpunktes des Acylphenols, vorzugsweise im Bereich von 40 bis 60 °C, werden die Feststoffe abgetrennt, vorzugsweise durch Filtrieren ; gegebenenfalls wird dann das Lösemittel entfernt, vorzugsweise durch Abdampfen, und das Reaktionsprodukt wird isoliert und gereinigt. Der Reinheitsgrad wird gaschromatographisch ermittelt ; er beträgt mindestens 92 %, vorzugsweise mindestens 98 %. Die Ausbeute liegt im allgemeinen bei mindestens 90 %, vorzugsweise mindestens 95 % der Theorie (bezogen auf reines Produkt).

Die erfindungsgemäß erhaltenen 2-Alkylphenole eignen sich zur Synthese von Wirkstoffen mit bioziden Eigenschaften ; sie werden auch zur Herstellung von pharmazeutischen Präparaten sowie von Konservierungs- und Desinfektionsmitteln verwendet.

Die nachstehenden Beispiele erläutern die Erfindung. Prozentangaben beziehen sich jeweils auf das Gewicht.

### Beispiel 1

In einem 2-Liter-Rührautoklav werden 200 g 2-Caproylphenol, 800 ml Ethanol und 20 g eines handelsüblichen Kupferkatalysators, der 60 % Kupfer auf Siliciumdioxid als Träger enthält, vorgelegt. Der Autoklav wird mit Stickstoff gespült und dann mit Wasserstoff bis zu einem Druck von 20 bar gefüllt. Der Autoklavinhalt wird 90 min lang auf 150 °C erhitzt, wobei der Wasserstoffdruck auf etwa 35 bar gehalten wird. Danach wird das Reaktionsgemisch auf 60 °C abgekühlt und filtriert, und das Filtrat wird bei einem Druck von 20 mbar eingeengt. Man erhält 182 g (= 98,1 % der Theorie) 2-Hexylphenol mit einer Reinheit von 95,1 %. Nach Destillation bei einem Druck von 10 mbar und einer Temperatur von 130 bis 132 °C resultiert ein farbloses Produkt.

### Beispiel 2

Beispiel 1 wird wiederholt mit 4-Butanoylresorcin als Ausgangsmaterial und Methanol als Lösemittel, wobei die Reaktionszeit 70 min und der Wasserstoffdruck 40 bar beträgt. Man erhält 186 g (= 100 % der Theorie) 4-Butylresorcin mit einer Reinheit von 95,8 %. Nach Destillation bei einem Druck von 10 mbar und einer Temperatur von 166 bis 168 °C resultiert ein farbloses, kirstallisierendes Produkt mit einem Schmelzpunkt von 47 bis 48 °C.

### Beispiel 3

Beispiel 1 wird wiederholt mit 4-(2-Ethylcaproyl)-resorcin als Ausgangsmaterial und Methanol als Lösemittel, wobei die Reaktionszeit 100 min beträgt. Man erhält 188 g (= 99,8 % der Theorie) (±)-4-(2-Ethylhexyl)-resorcin mit einer Reinheit von 99,2 %. Nach Destillation bei einem Druck von 10 mbar und einer Temperatur von 182 bis 185 °C resultiert ein farbloses, kristallisierendes Produkt mit einem Schmelzpunkt von 48 bis 50 °C.

## Ansprüche

1. Verfahren zur Herstellung eines 2-Alkylphenols durch Reduktion eines 2-Acylphenols in Gegenwart eines metallischen Katalysators bei einem Druck von 10 bis 100 bar, gegebenenfalls in Gegenwart eines inerten Lösemittels, dadurch gekennzeichnet, daß bei einer Temperatur von 100 bis 160 °C eine Verbindung der Formel (1)

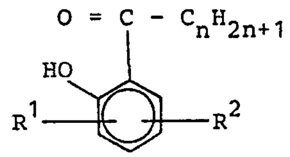

(1)

hydriert wird, in der n eine ganze Zahl von 2 bis 17 darstellt und $R^1$ und $R^2$ gleich oder verschieden sind und jeweils ein Wasserstoffatom, ein Halogenatom, eine Hydroxylgruppe, eine Alkoxygruppe mit 1 bis 16 Kohlenstoffatomen, eine Acylgruppe oder Acyloxygruppe mit jeweils 2 bis 10 Kohlenstoffatomen, eine Dialkylaminogruppe mit insgesamt 2 bis 14 Kohlenstoffatomen oder einen Kohlenwasserstoffrest mit 1 bis 16 Kohlenstoffatomen bedeutet, und als Katalysator Kupfer, Nickel oder Kobalt in einer Menge von 0,5 bis 25 Gewichtsprozent (bezogen auf das 2-Acylphenol) verwendet wird.

2. Verfahren nach Anspruch 1, dadurch gekennzeichnet, daß der Katalysator in Verbindung mit einem Träger verwendet wird.

3. Verfahren nach Anspruch 2, dadurch gekennzeichnet, daß das Gewichtsverhältnis von Katalysator zu Träger 1 : 0,2 bis 1 : 10 beträgt.

4. Verfahren nach Anspruch 2, dadurch gekennzeichnet, daß als Träger Siliciumdioxid oder Aluminiumoxid verwendet wird.

## Claims

1. Process for the preparation of a 2-alkylphenol by reduction of a 2-acylphenol in the presence of a metallic catalyst under a pressure of 10 to 100 bars, if desired in the presence of an inert solvent, characterized in that a compound of the formula (1)

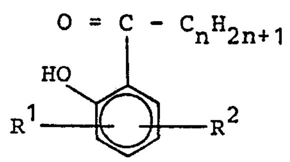

(1)

in which n represents an integer from 2 to 17 and $R^1$ and $R^2$ are identical or different and each means a hydrogen atom, a halogen atom, a hydroxyl group, an alkoxy group having 1 to 16 carbon atoms, an acyl or acyloxy group each having 2 to 10 carbon atoms, a dialkylamino group having a total of 2 to 14 carbon atoms or a hydrocarbon radical having 1 to 16 carbon atoms, is hydrogenated at a temperature of 100 to 160 °C and that as catalyst there is used copper, nickel or cobalt in an amount of from 0.5 to 25 percent by weight (calculated on the 2-acylphenol).

2. Process according to claim 1, characterized in that the catalyst is used in connection with a support.

3. Process according to claim 2, characterized in that the weight ratio of catalyst to support is from 1 : 0.2 to 1 : 10.

4. Process according to claim 2, characterized in that as support there is used silicon dioxide or aluminium oxide.

## Revendications

1. Procédé de préparation d'un alkyl-2 phénol par réduction d'un acyl-2 phénol en présence d'un catalyseur métallique, sous une pression de 10 à 100 bar, éventuellement en présence d'un solvant inerte, procédé caractérisé en ce qu'on hydrogène, à une température de 100 à 160 °C, un composé répondant à la formule (1)

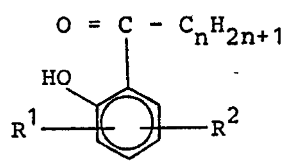

(1)

dans laquelle n désigne un nombre entier de 2 à 17, et $R^1$ et $R^2$ représentent chacun, indépendamment l'un de l'autre, un atome d'hydrogène ou d'halogène, un radical hydroxy, un radical alcoxy contenant de 1 à 16 atomes de carbone, un radical acyle ou un radical acyloxy contenant chacun de 2 à 10 atomes de carbone, un radical dialkylamino contenant au total de 2 à 14 atomes de carbone ou un radical hydrocarboné contenant de 1 à 16 atomes de carbone, et en ce qu'on utilise, comme catalyseur, du cuivre, du nickel ou du cobalt en une quantité de 0,5 à 25 % en poids (par rapport à l'acyl-2 phénol).

2. Procédé selon la revendication 1, caractérisé en ce que le catalyseur est utilisé en association avec un support.

3. Procédé selon la revendication 2, caractérisé en ce que le rapport pondéral du catalyseur au support est compris entre 1 : 0,2 et 1 : 10.

4. Procédé selon la revendication 2, caractérisé en ce qu'on utilise, comme support, de la silice ou de l'alumine.